**Europäisches Patentamt**

**European Patent Office**

**Office européen des brevets**

(19)

(11) Veröffentlichungsnummer: **0 002 560**

**A1**

# (12) EUROPÄISCHE PATENTANMELDUNG

(21) Anmeldenummer: **78200377.6**

(22) Anmeldetag: **18.12.78**

(51) Int. Cl.²: **A 61 B 6/02**
**A 61 B 6/06**

(30) Priorität: **19.12.77 DE 2756659**

(43) Veröffentlichungstag der Anmeldung:
**27.06.79 Patentblatt 79/13**

(84) Benannte Vertragsstaaten:
**BE DE FR GB IT NL SE**

(71) Anmelder: **Philips Patentverwaltung GmbH**
**Steindamm 94**
**D-2000 Hamburg 1(DE)**

(84) Benannte Vertragsstaaten:
**DE**

(71) Anmelder: **N.V. Philips' Gloeilampenfabrieken**
**Prof. Holstlaan 6**
**Eindhoven(NL)**

(84) Benannte Vertragsstaaten:
**BE FR GB IT NL SE**

(72) Erfinder: **Lux, Peter, Dr.**
**Busken Huetstraat 53**
**Eindhoven(NL)**

(74) Vertreter: **Poddig, Dieter et al,**
**Philips Patentverwaltung GmbH Steindamm 94**
**D-2000 Hamburg 1(DE)**

(54) **Anordnung zur Bestimmung der Absorptionsverteilung.**

(57) Bei der Bestimmung der Absorptionsverteilung in einer Ebene eines Körpers (4) mit Hilfe eines Fächers von Strahlenbündeln (5) aus einer annähernd punktförmigen Strahlungsquelle (1) und verschiedener Positionen des Körpers (4) relativ zur Strahlungsquelle wird ein einziges Detektorelement (6) fur alle Strahlenbundel (5) verwendet. Zur Bestimmung des Intensitätsprofils in jeder Messposition werden die Strahlenbundel (5) mit Zeitfunktionen moduliert, die ein vollständiges, linear unabhängiges Funktionssystem bilden, (Walsh-Funktionen). Diese Modulation erfolgt mittels einer Blende (3), die als rotierende Scheibe ausgeführt ist, die durchlässige und undurchlässige Sektoren (S1-S4) besitzt. Aus den Detektorsignalen wird bei jedem Wechsel mindestens eines Funktionswertes, ein diskreter Signalwert entnommen, so dass die Gesamtzahl der Signalwerte gleich der Anzahl der Funktionen des Funktionssytems ist. Diese Signalwerte werden mit einer Rücktransformationsmatrix multipliziert und daraus wird das Intensitätsprofil gewonnen. Dieses Prinzip eignet sich auch für einen zweidimensionalen Fächer von Strahlenbündeln, um die Absorptionsverteilung in mehreren Ebenen gleichzeitig zu bestimmen.

FIG.1

"Anordnung zur Bestimmung der Absorptionsverteilung".


Die Erfindung betrifft eine Anordnung zur
Bestimmung der Absorptionsverteilung eines Körpers
mittels einer im wesentlichen punkförmigen Quelle zur
Erzeugung von den Körper durchdringenden Strahlung und
einer Detektoranordnung, die die den Körper durchdrungene Strahlung aufnimmt und in ein Detektor-Signal
umwandelt, und einer Verarbeitungsanordnung, die aus
den Detektorsignalen elektrische Signale erzeugt, die
die Absorption des Körpers längs benachbarter Strahlenbündel angeben.
Eine derartige Anordnung, die insbesondere
in der medizinischen Röntgentechnik verwendet wird, ist
beispielsweise aus der DT-OS 24 39 847 bekannt. Von der
Strahlung, die die strahlenquelle erzeugt, werden fächerförmige Strahlenbündel ausgeblendet, die auf den zu
untersuchenden Körper gerichtet sind. Auf der entgegengesetzten Seite des Körpers ist eine Detektoranordnung
aus einem Detektorelement für jedes Strahlenbündel
angeordnet. Jedes Detektorelement besteht aus einem
Scintillationskristall mit einem Fotovervielfacher und
einem daran angeschlossenen Verstärker. Die Verstärkung
der Verstärker muss individuell auf die Empfindlichkeit
der Kristalle und der Fotovervielfacher eingestellt
werden, wobei die Kette Kristall-Fotovervielfacher-Verstärker eine für alle Detektorelemente möglichst
genau gleiche Umsetzung von Strahlung in elektrisches
Signal liefern muss, damit reproduzierbare Ergebnisse
erhalten werden können. Dies erfordert eine sehr aufwendige
und sorgfältige Einstellung der einzelnen Verstärker,
die ausserdem in gewissen Zeitabständen wegen der Alterung
der verschiedenen Elemente wiederholt werden muss.
Diese bekannte Anordnung erfordert daher, abgesehen von
dem sehr hohen Herstellungsaufwand in Form einer grossen
Anzahl Fotovervielfacher und Verstärker, auch einen hohen
Aufwand im Betrieb durch die ständig erforderliche Überwachung und Justierung durch Fachpersonal.

　　　　Aufgabe der vorliegenden Erfindung ist es,
eine Anordnung anzugeben, mit der die Absorptionsverteilung bei geringerem Aufwand sowohl in der Herstellung
wie auch im Betrieb bestimmt werden kann.

　　　　Diese Aufgabe wird erfindungsgemäss dadurch
gelöst, dass die Detektoranordnung für jeweils eine Gruppe
benachbarter Strahlenbündel nur ein Detektorelement enthält, dass eine Modulationsanordnung die Intensität
der Strahlenbündel nach Zeitfunktionen moduliert, die
jeweils für die von einem Detektorelement aufgenommenen
Strahlenbündel ein vollständiges, linear unabhängiges
Funktionssystem bilden, bei dem jede Funktion eindeutig
einem Strahlenbündel zugeordnet ist, wobei die Funktionen
nur positive Funktionswerte beinhalten, und dass die
Verarbeitungsanordnung aus den Detektorsignalen eine
Anzahl vorzugsweise in etwa gleichen Abständen liegende
diskrete Signalwerte entnimmt, und speichert, wobei die
Anzahl der diskreten Signalwerte gleich der Anzahl der
Funktionen des zugehörigen Funktionssystems ist, und
durch Multiplikation der diskreten Signalwerte mit einer
Matrix von Werten, die zu der Matrix von Werten invers
ist, die die Funktionswerte der beim Modulieren verwendeten Funktionen an den der Entnahme der diskreten
Signalwerte entsprechenden Stellen darstellen, und
nachfolgende Logarithmierung die die Absorption angebenden
Signalwerte erzeugt.

Dadurch ist für jeweils eine Anzahl Strahlenbündel beispielsweise nur ein Scintillationskristall
oder ein aus mehreren Teilen zusammengesetzter Kristall,
nur ein Fotovervielfacher und nur ein Verstärker notwendig.
Eine Veränderung der Empfindlichkeit, d.h. der Umsetzcharakteristik von Strahlung in elektrisches Signal,
betrifft dabei dann eine grössere Anzahl paralleler
Strahlenbündel gleichmässig, so dass keine Verfälschung
durch gegenseitige Verschiebung der Messsignale gegeneinander entstehen kann, sondern alle Signale werden
gleichmässig beeinflusst, als würde die Intensität der
Strahlungsquelle verändert. Dies gilt für alle Arten
von Detektorelementen, beispielsweise aus Xenon-
Ionisationskammern. Eine Veränderung während eines
Messvorganges wird je nach dem verwendeten Funktionssystem mehr oder weniger über die gesamten Messwerte
verteilt und tritt dadurch nur mit stark verminderter
Amplitude in Erscheinung.

Die diskreten Signalwerte, die die Verarbeitungsanordnung den Detektorsignale entnimmt, sollen bestimmten
Funktionswerten der beim Modulieren verwendeten Funktionen
entsprechen, d.h. Augenblickswerte darstellen. Aus verschiedenen Gründen sind solche Augenblickswerte jedoch
fehlerbehaftet, beispielsweise durch das starke Rauschen,
das bei insbesondere im medizinischen Anwendungsbereich
verwendeten geringen Strahlungsdosen auftritt. Aus diesem
Grunde ist es zweckmässig, dass die Modulationsanordnung
jedes Strahlenbündel mit nur diskreten Funktionswerten
der zugehörigen Funktionen während jeweils einer vorgegebenen Zeitdauer moduliert, wobei die Anzahl der diskreten Funktionswerte gleich der Anzahl der verwendeten
Funktionen des zugehörigen Funktionssystems ist und die
Funktionswerte in etwa gleichen zeitlichen Abständen
aus den Funktionen entsprechend den Stellen der Entnahme
der diskreten Signalwerte in der Verarbeitungsanordnung
entnommen sind. Während der Zeitdauer der Modulation mit
einem diskreten Funktionswert wird das von der Detektoranordnung abgegebene elektrische Signal integriert, wodurch insbesondere der Einfluss des Rauschens stark zurückgeht, und erst am Ende dieser Zeitdauer wird der diskrete Signalwert von der Verarbeitungsanordnung entnommen. Zwischen den einzelnen Zeitdauern der Modulation können Pausen eingeschoben werden, in denen die gesamte Strahlungsintensität 0 ist.

Die Absorptionswerte längs benachbarter Strahlenbündel bilden eine Absorptionsfunktion mit Sequenzen verschiedener Ordnung bis maximal der Anzahl der Strahlenbündel, die von dem Detektorelement aufgenommen werden. Es hat sich herausgestellt, dass visuelle Bildeindruck nicht verfälscht wird, wenn die höheren Sequenzen in dieser Absorptionsfunktion stärker rauschbehaftet sind. Um die Gesamtdosis bei der Durchstrahlung des Körpers zu verringern, ist es daher zweckmässig, dass die Modulationsanordnung die Strahlenbündel mit den zugehörigen Funktionswerten derjenigen Funktionen, die die höheren Sequenzen der durch die Folge der Absorptionwerte längs benachbarter Strahlenbündel gebildeten Absorptionsfunktion erzeugen, während kürzerer Zeitdauer moduliert als mit den Fuktionswerten der übrigen Funktionen und während der restlichen Zeit diese Strahlenbündel ganz sperrt. Wenn bei der Modulation mit diskreten Funktionswerten zwischen aufeinanderfolgende Funktionswerte jeweils eine Pause eingeschoben wird, wird diese Pause bei den Funktionen für die höheren Sequenzen entsprechend verlängert.

Eine weitere Möglichkeit für die Verringerung der beim Untersuchen des Körpers verwendeten Strahlungsdosis besteht darin, dass in den Funktionssystemen die Funktion weggelassen ist, die den Gleichanteil, d.h. den Mittelwert der Absorptionswerte für die Strahlenbündel eines Detektorelementes, liefert. Dieser Gleichanteil wird nämlich durch den Filterprozess beim Rekonstruieren der zweidimensionalen Absorptionsverteilung ohnehin eliminiert. Für die Multiplikation der diskreten Signalwerte mit der Matrix von Funktionswerten kann dann ein angenommener Gleichanteil eingesetzt werden, dessen Wert zweckmässig so gross gewählt wird, dass bei
der Multiplikation keine negativen Werte entstehen.

Zweckmässig ist es, wenn für alle Strahlenbündel
nur ein Detektorelement vorhanden ist. In diesem Falle
wird auch nur ein Verstärker benötigt, der leicht eingestellt werden kann bzw. dessen langzeitbedingten
Verstärkungsänderungen nur die gesamte Bildhelligkeit
gleichmässig verändern.

Bisher wurde von nur in einer Dimension einen
Fächer bildenden Strahlenbündel ausgegangen. Wenn mehrere
parallele Schichten, die eine dreidimensionale Information
über die Absorptionsverteilung des Körpers liefern,
untersucht werden sollen, werden die Absorptionswerte
der einzelnen Schichten allgemein nacheinander bestimmt.
In Ausgestaltung der vorliegenden Erfindung ist es jedoch
zweckmässig, dass die Strahlenbündel einen zweidimensionalen Fächer bilden und jeweils für nur in einer Dimension
benachbarte Strahlenbündel ein Detektorelement vorgesehen
ist. Wenn jeweils für alle Strahlenbündel einer Ebene
ein Detektorelement vorhanden ist, wird dann für jede
zu untersuchende Schicht ein Detektorelement benötigt.
Die in der anderen Dimension übereinander liegenden
Strahlenbündel werden dann mit gleichen Funktionen
moduliert, und die Verarbeitungsanordnung behandelt die
elektrischen Signale die einzelnen Detektorelemente in
gleicher Weise, z.B. zeitlich parallel.

Bei einem zweidimensionalen Fächer ist es in
konsequenter Anwendung des Erfindungsgedankens auch
zweckmässig, dass für alle Strahlenbündel nur ein
Detektorelement vorhanden ist und die Modulationsanordnung die Strahlenbündel nach Zeitfunktionen moduliert,
die ein zweidimensionales, vollständiges, linear unabhängiges Funktionssystem bilden. In diesem Fall muss die
Verarbeitungsanordnung die diskreten Signalwerte zur
Rücktransformation mit einer dreidimensionalen Matrix
multiplizieren bzw. eine zweiseitige Multiplikation mit
zweidimensionalen Matrizen durchführen, um eine zweidimensionale Matrix von Absorptionssignalen zu erzeugen.

Es sind verschiedene vollständige, linear unabhängige Funktionssysteme bekannt, beispielsweise das bei der Fourier-Analyse verwendete System der Sinus- und Kosinus-Funktionen. Die Verwendung eines solchen Funktionssystems erfordert jedoch eine Modulation mit einer Vielzahl von unterschiedlichen Werten, die Schwierigkeiten bereiten kann. Besonders zweckmässig ist es daher, dass das Funktionssystem bzw. die Funktionssysteme das System der Walsh-Hadamard-Funktionen ist. Bei diesen Funktionen bzw. den entsprechend verschobenen Funktionen kommt dann nur der Wert 1 oder 0 beim Modulieren zur Wirkung, d.h. ein Strahlenbündel wird ganz durchgelassen oder ganz gesperrt, was ohne Schwierigkeiten möglich ist. Andere Funktionssysteme, die nur zwei verschiedene Werte annehmen können, sind die Haar-Funktionen sowie die m-Funktionen, die in der Zeitschrift "Frequenz", Heft 7 (1976), Seite 196 bis 199 beschrieben sind.

Es ist zweckmässig, dass die Modulationsanordnung eine mechanisch bewegte Blende zwischen der Quelle der Strahlung und der Detektoranordnung ist, die entsprechend den Funktionen der Funktionssysteme für die Strahlung durchlässige bzw. undurchlässige Bereiche besitzt. Eine derartige Modulationsanordnung ist für die verschiedensten Arten von Strahlenquellen brauchbar, insbesondere auch für Röntgenstrahlen, und sie ist leicht herzustellen. Dabei ist es zweckmässig, dass die Blende aus einer oder mehreren rotierenden Scheiben besteht. Mit einer solchen Ausführung der Blenden ist auch eine relativ schnelle Bewegung leicht zu erreichen.

Die von der Verarbeitungsanordnung entnommenen diskreten Signalwerte aus den Detektorsignalen sollen möglichst genau bestimmten Funktionswerten bzw. bestimmten Zeitpunkten beim Modulieren mit diskreten Funktionswerten entsprechen. Es ist daher zweckmässig, dass von der Bewegung der Blende Synchronisiersignale zum Steuern der Entnahme der diskreten Signalwerte in der Verarbeitungsanordnung abgeleitet sind. Auf diese Weise wird eine sichere Zuordnung der entnommenen diskreten Signalwerte zu bestimmten Funktionswerten bzw. Zeitpunkten gewährleistet.

Ausführungsbeispiele der Erfindung werden nachstehend anhand der Zeichhung erläutert. Es zeigen:

Fig. 1 eine schematische Gesamtansicht der
Anordnung,

Fig. 2 ein Beispiel für eine scheibenförmige
Blende mit Anwendung der Walsh-Hadamard-Funktionen,

Fig. 3 ein anderes Beispiel einer Blende mit
Anwendung der m-Funktionen,

Fig. 4 ein Beispiel einer Blende für ein zweidimensionales Walsh-Hadamard-Funktionssystem,

Fig. 5 eine schematische Gesamtansicht einer
erweiterten Anordnung nach Fig. 1,

Fig. 6 ein Blockschaltbild der Verarbeitungsanordnung.

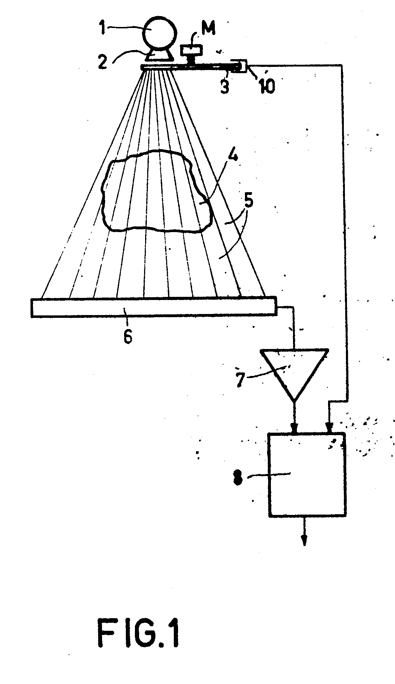

In Fig. 1 erzeugt eine im wesentlichen punktförmige Quelle die Strahlung, mit der der Körper 4 untersucht werden soll. Diese Quelle kann beispielsweise eine
Röntgenröhre sein. Vor dieser Strahlungsquelle ist ein
Kollimator 2 angeordnet, der von der erzeugten Strahlung
einen in der Zeichnungsebene liegenden Fächer von Strahlenbündel 5 ausblendet.

Vor dem Kollimator 2 ist eine Blende 3 in Form
einer rotierenden Scheibe angeordnet, die von einem Motor
M angetrieben wird. Diese Blende 3 moduliert die einzelnen
Strahlenbündel nach unterschiedlichen Zeitfunktionen.

Die Strahlenbündel 5 treffen auf den zu untersuchenden Körper 4 und werden beim Durchgang durch diesen
Körper entsprechend dem Integral der Absorption des
Körpers längs des strahlanbündels abgeschwächt.

Die den Körper 4 durchdringende Strahlung
trifft auf eine Detektoranordnung 6, die hier aus einem
einzigen Detektorelement besteht. Dieses Detektorelement
kann aus einem oder mehreren Scintillationskristallen
bestehen, deren Lichtblitze von einem einzigen Fotovervielfacher aufgenommen werden, oder aus einer Xenon-
Messkammer, und es erzeugt ein elektrisches Signal, das der auf dem Detektor 6 auftreffenden gesamten
Strahlung entspricht.

Das elektrische Detektorsignal wird in einem
Verstärker 7 verstärkt, dessen Verstärkung zweckmässig
auf einen bestimmten Umwandlungswert von Strahlung in
elektrisches Signal eingestellt ist, so dass der eigentlichen Verarbeitungsanordnung 8 ein normiertes Signal
zugeführt wird. Die Verarbeitungsanordnung 8 erzeugt
aus diesem Signal mit Hilfe des von der Bewegung der
Blende 3 mittels Positionsdetektor 1o abgeleiteten
Synchronisiersignals nacheinander Signale, die die
Absorption des Körpers 4 längs der einzelnen Strahlenbündel in der Reihefolge ihrer Lage in der Zeichnung
angeben.

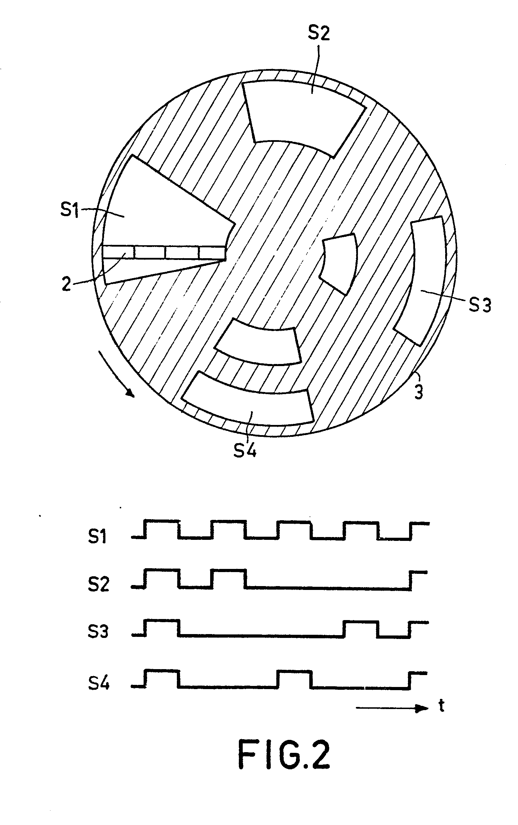

Zur Erläuterung der Wirkungsweise dieser
Anordnung wird angenommen, dass die Blende 3 die einzelnen
Strahlenbündel nach Walsh-Funktionen moduliert. Eine dafür
geeignete rotierende Blende ist in Fig. 2 dargestellt.
Diese Blende besitzt 4 Sektoren S1 bis S4 mit Durchbrüchen, die für die Strahlung durchlässig sind, während
die schraffierten Bereiche undurchlässig sind. Die 4
Sektoren sind durch undurchlässige Abschnitte getrennt.
Der vor der Quelle der Strahlung angeordnete Kollimator 2
befindet sich in der gezeichneten Lage der Blende 3 gerade
hinter dem Sektor S1. In Fig. 2 ist eine Unterteilung des
Kollimators in 4 Abschnitte dargestellt, die tatsächlich
nicht vorhanden zu sein braucht und die 4 nebeneinanderliegende Strahlenbündel andeuten soll. Bei Drehung in
Pfeilrichtung werden die 4 Strahlenbündel in einer Weise
zeitlich moduliert, wie unter der Blende in dem Zeitdiagramm mit t angegeben ist. Wenn in diesem Diagram
aus den 4 Kurvenzügen die Teile herausgenommen werden,
die durch die undurchlässigen Bereiche zwischen den
Sektoren der Blende erzeugt werden, ergeben sich gerade
die 4 ersten Walsh-Funktionen, deren Werte der Hadamard-
Matrix vierter Ordnung entsprechen:

$$\left[H_4\right] = \begin{vmatrix} 1 & 1 & 1 & 1 \\ 1 & 1 & -1 & -1 \\ 1 & -1 & -1 & 1 \\ 1 & -1 & 1 & -1 \end{vmatrix}$$

Bei diesem Vergleich ist natürlich vorausgesetzt, dass die Funktionswerte -1 beim Modulieren durch den Wert 0 ersetzt werden, da es negative Strahlung in diesem Sinne nicht gibt. Die zum Modulieren verwendeten Funktionen $M_i$ (x), wobei x die Werte von 0 bis 1 annimmt und die Winkeldrehung der Blende bei einem Umlauf und damit die Zeit angibt, sind also auf folgende Weise von den Walsh-Funktionen abgeleitet:

$$M_i \left(x\right)_o^1 = \tfrac{1}{2}\left[Wal \left(i,x\right) + Wal \left(0,x\right)\right]$$

Wenn das Ausgangssignal des Detektorelements 6 bzw. des Verstärkers 7 während der Zeit integriert wird, während sich jeweils ein Sektor der Blende 3 vor dem Kollimator 2 befindet, und am Ende dieser Zeit abgetastet wird, geben diese Signale die wegen der Modulation mit verschobenen Walsh-Funktionen modifizierten Spektralwerte $F_w'$ (i) an, die mit den Walsh-Spektralwerten in folgender Weise zusammenhängen.

$$F_w' \left(i\right)_o^1 = \tfrac{1}{2}\left[F_w \left(i\right) + F_w \left(o\right)\right].$$

Die unmodifizierten Walsh-Spektralwerte $F_w$ (i) ergeben sich daraus zu

$$F_w \left(i\right) = 2\, F_w' \left(i\right)_o^1 - F_w \left(o\right)_o^1.$$

In diesen Gleichungen bedeutet i jeweils die Ordnungsnummer der Strahlenbündel, und zwar bei der in Fig. 2 dargestellten Blende von links mit 0 beginnend gezählt. Aus den so erhaltenen, unmodifizierten Walsh-Spektralwerten kann die Funktion der räumlichen Verteilung der Strahlungsintensität auf dem Detektorelement dadurch bestimmt werden, dass diese Spektralwerte mit einer Matrix multipliziert werden, die zu der beim Modulieren verwendeten Matrix invers ist. Im Falle der Walsh-Hadamard-Funktionen ist dies die gleiche, bereits angegebene Hadamard-Matrix vierter Ordnung. Aus der so gewonnenen Intensitätsverteilung kann dann in bekannter Weise durch Logarithmieren die Absorptionsverteilung, d.h. das Integral der Absorption des Körpers längs der einzelnen Strahlenbündel, bestimmt werden.

Nachfolgend soll dieser Ablauf anhand eines Zahlenbeispiels erläutert werden, wobei angenommen wird, dass der Körper eine derartige Absorptionsverteilung hat, dass die von der Blende durchgelassenen Strahlenbündel von links nach rechts gezählt mit (hier relativ angegebenen) folgenden Intensitäten beim Detektorelement auftreffen: 1; 2; 3; 4. Bei der Drehung der Blende, beginnend mit dem Sektor S1, gibt der Detektor dann nacheinander Signale entsprechend den folgenden Gesamtintensitäten ab: 10; 3; 5; 4. Um von diesen modifizierten Spektralwerten zu den ursprünglichen Spektralwerten der räumlichen Intensitätsverteilung der auf den Detektor auftreffenden Strahlung aus allen Strahlenbündeln zu kommen, wird die letztgenannte Gleichung angewendet und ergibt die folgenden Werte: 10; -4; 0; -2. Wenn diese Spektralwerte als einspaltige Matrix mit der Hadamard-Matrix vierter Ordnung multipliziert wird, ergeben sich die folgenden Werte: 4; 8; 12; 16.

Dies sind bis auf den Normierungsfaktor 4 die Intensitäten der Strahlenbündel beim Auftreffen auf das Detektorelement, und zwar in gleicher Reihenfolge.

Die Anzahl von vier Strahlenbündeln ist in dem vorstehend beschriebenen Beispiel nur der Übersichtlichkeit halber gewählt worden. In der Praxis ist selbstverständlich eine höhere Auflösung notwendig, die eine sehr viel grössere Anzahl von Strahlenbündeln und damit von verwendeten Funktionen erfordert. Das System der Walsh-Funktionen ist bis zu beliebig hohen Ordnungen definiert. Die Blende 3 enthält dann eine entsprechend grössere Anzahl von Sektoren, nämlich gleich der Anzahl der verschiedenen Strahlenbündel, und die Sektoren sind dann entsprechend den Walsh-Funktionen feiner unterteilt.

Bei den Walsh-Funktionen sind die diskreten Funktionswerte zu bestimmten Augenblicken gleich den Funktionswerten für einen bestimmten Abschnitt, und diese diskreten Funktionswerte können auch nur zwei verschiedene Zahlenwerte annehmen, so dass die Ausbildung der Blende 3 hier besonders einfach ist. Grundsätzlich können aber auch andere vollständige, linear unabhängige Funktionssysteme verwendet werden, die beispielsweise kontinuierliche Funktionen enthalten. Beim Modulieren mit dem gesamten Funktionsverlauf solcher kontinuierlicher Funktionen kann bei Verwendung einer Blende diese an den Trennstellen zwischen jeweils zwei Strahlenbündel einen Steg enthalten, dessen Breite auf beiden Seiten des Strahlenbündels eine Breite entsprechend der verwendeten Funktion besitzt. Die Trennabschnitte zwischen den Sektoren können dabei, ebenso wie auch bei den beschriebenen Walsh-Funktionen, ganz weggelassen werden, wenn dies die Stabilität der Blende zulässt. Die Blende kann aber auch im Sinne eines Filters aufgebaut sein, die Bereiche unterschiedlicher Durchlässigkeit entsprechend den verwendeten Funktionen aufweist.

Die vorstehend beschriebene Blende mit den Walsh-Hadamard-Funktionen muss bei jeder relativen Winkelstellung des Körpers 4 zu der Anordnung 1 - 6 soweit gedreht werden, dass alle Werte aller Funktionen gerade einmal an dem Kollimator 2 vorbeigeführt worden sind, d.h. um eine Umdrehung, wenn diese Funktionen auf dem gesamten Umfang der Blende verteilt sind. Eine andere Möglichkeit, die eine geringere Drehzahl der Blende 3 erfordert, ergibt sich bei der Verwendung der m-Funktionen.

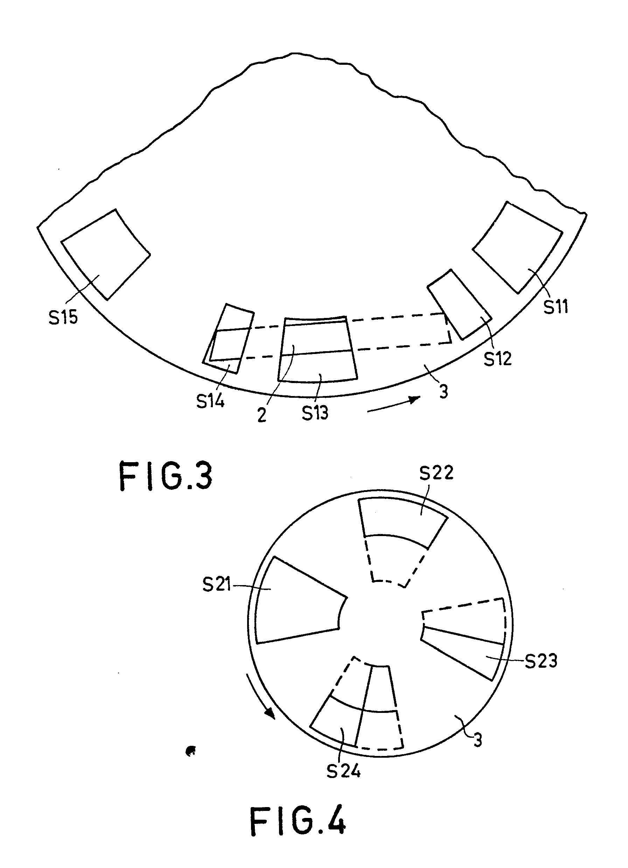

Eine dafür geeignete Blende ist in Fig.3 dargestellt. Auch diese Blende ist wieder als rotierende Scheibe ausgebildet, wobei die einzelnen Funktionswerte der verschiedenen Funktionen, die beim Modulieren der Strahlen gleichzeitig wirksam werden, nicht mehr radial, sondern auf dem Umfang verteilt angeordnet sind. Wie aus der eingangs erwähnten Literaturstelle "Frequenz", Heft 7 (1967), Seite 196 bis 199 hervorgeht, entsteht
jede Funktion dadurch aus der vorhergehenden Funktion,
dass diese zyklisch um ein Teilintervall verschoben wird.
Auf dem Umfang der Scheibe sind daher mehrere Folgen der
gleichen Funktion aufeinanderfolgend angebracht, wobei
jede Funktion zwei durchlässige Sektoren enthält, d.h.
eine Funktion möge vom Beginn des Sektors S11 bis zum
Beginn des Sektors S13 reichen und den Sektor S12 mit
umfassen. Die anschliessende, gleiche Funktion reicht
vom Beginn des Sektors S13 bis zum Beginn des Sektors S15
und umfasst den Sektor S14 mit, usw.. Von der Blende 3
ist in Fig. 3 daher nur ein Teil dargestellt, aus dem
diese Folge der Funktionen deutlich wird.

Sobald diese Blende 3 sich in Pfeilrichtung
um die Breite des Sektors S12 bewegt hat, werden die
aus dem Kollimator 2 austretenden Strahlen mit der
nächsten Funktion moduliert. Bei jeder Position des
Körpers 4 in Fig. 1 muss die Blende 3 also nur so weit
bewegt werden, dass sich alle Funktionen einmal vor
dem Kollimator befinden, d.h. um den Winkel, den eine
vollständige Funktion auf dem Umfang besetzt.

Die Begrenzung der vor den Enden des Kollimators 2
liegenden Sektoren, in der in Fig. 3 dargestellten Stellung
insbesondere die Begrenzung des Sektors S14, stehen nicht
senkrecht zur Richtung des Kollimators, jedoch ergibt
dies nur eine geringe Verschiebung der dadurch erzeugten
Werte. Diese Verschiebung wird noch geringer, wenn die
Blende bzw. das Verhältnis zwischen der Entfernung des
Kollimators 2 vom Drehpunkt der Blende 3 zur Länge des
Kollimators 2 grösser ausgeführt wird, so dass eine
vollständige Funktion einen kleineren Winkelbereich
auf der Blende 3 einnimmt und entsprechend mehr Funktionen
auf dem Umfang angeordnet werden. Eine weitere Verschiebung der Werte entsteht noch dadurch, dass die Blende 3
kontinuierlich bewegt wird und dabei ein fliessender
Übergang von einer Funktion auf die nächste erfolgt.
Dies stellt im vorliegenden Falle eine Art "Übersprechen"
zwischen benachbarten Strahlenbündeln und damit zwischen benachbarten Intensitätswerten dar, was die gleiche Wirkung hat, als wenn bei der bekannten Verwendung von einzelnen Detektorelementen je Strahlenbündel sich die räumlichen Aufnahmebereiche der einzelnen Detektoren überlappen. Dieser Effekt ist durchaus erwünscht, da er ein sogenanntes "fold back", d.h. ein Umklappen höherer Sequenzanteile in niedrigere Sequenzbereiche verhindert, die durch Details entstehen, deren Ausdehnung kleiner als die eines Strahlenbündels ist.

Es sei darauf hingewiesen, dass die Blenden auch andere Formen haben können, beispielsweise als Walze ausgebildet sein können, wobei die Quelle 1 der Strahlung und der Kollimator 2 im Inneren angeordnet sind.

Bei Verwendung der beschriebenen Anordnung in einer Röntgenanlage für medizinische Zwecke ist es erwünscht, mit einer möglichst geringen Strahlungsdosis zu arbeiten. Die unterste Grenze der Strahlungsdosis wird dadurch bestimmt, dass die von der Strahlung die vom Detektorelement 6 aufgenommen wird, erzeugten Signale genügend gross gegenüber dem von dem Detektorelement und dem angeschlossenen Verstärker erzeugten Rauschen sind. Es hat sich nun herausgestellt, dass der visuelle Eindruck des Bildes, das die aus den Absorptionswerten in verschiedenen relativen Stellungen der Anordnung zu dem zu untersuchenden Körper gewonnene Absorptionsverteilung in der Untersuchungsebene zeigt, nicht verfälscht wird, wenn die höheren Sequenzen der Absorptionsfunktion stärker rauschbehaftet sind. Da die gesamte Strahlendosis auch durch die Zeitdauer bestimmt wird, während der die Blende 3 die einzelnen Strahlenbündel durchlässt, können in dem Blendenbereich, der den Funktionen für die Erzeugung der höheren Sequenzen der Intensitätsfunktion erzeugt, die Blendensektoren der in Fig. 2 dargestellten Blende schmaler gewählt werden, vorzugsweise entsprechend der Drehrichtung der Blende später beginnend. Statt dessen können auch Stege in den Sektoren an den Rändern der Strahlenbündel angeordnet werden, die diesen Funktionen für die höheren Sequenzen zugeordnet sind. Bei Verwendung

0002560

von anderen als den Walsh-Funktionen können die Stege entsprechend verbreitert bzw. der Dämpfungsfaktor des Blendenbereiches erhöht werden. Die angegebene Massnahme zur Reduktion der Bestrahlungsdosis wirkt in jedem Falle so, als werden die Strahlenbündel für die höheren Sequenzen mit entsprechend geringerer Amplitude moduliert, als dem Funktionswert der zugehörigen Funktion entspricht. Diese Verringerung der Amplitude muss bei der Verarbeitung der aus dem Detektorsignal entnommenen diskreten Signalwerte berücksichtigt werden. Diese Möglichkeit zur Verringerung der Strahlungsdosis besteht jedoch nur bei Verwendung von solchen Funktionssystemen, die die Koeffizienten der einzelnen Sequenzen erzeugen, d.h. insbesondere bei Walsh-Funktionen, jedoch nicht bei den angegebenen m-Funktionen.

Eine weitere Verringerung der bei der Messung der Absorptionswerte verwendeten Strahlungsdosis kann dadurch erreicht werden, dass die Funktion in dem Funktionssystem bzw. der zugehörige Blendenteil, der den Gleichanteil der Absorptionswerte. d.h. die Summe der Intensitäten aller Strahlenbündel liefert, weggelassen wird. Bei dem in Fig. 2 dargestellten Beispiel einer Blende wäre denn der Sektor S1 undurchsichtig, und die Verarbeitungsanordnung 8 in Fig. 1 würde dann dem Ausgangssignal des Detektors 6 bzw. des Verstärkers 7 nur drei Funktionswerte entnehmen. Für den Gleichanteil kann ein Funktionswert willkürlich angenommen werden, beispielsweise kann er auch den Wert 0 haben, jedoch ist es zweckmässig, diesen willkürlichen Funktionswert so zu wählen, dass die rücktransformierten Werte vor der Logarithmierung in der Anordnung 8 nur positiv sind. Andererseits sollte dieser willkürlich gewählte Wert auch nicht zu gross sein, damit bei der Weiterverarbeitung der rücktransformierten Werte nicht unnötig viele Stellen verarbeitet werden müssen.

Wenn auf diese Weise die Absorptionswerte des Körpers 4 in verschiedenen Messpositionen des Körpers 4 relativ zu der Anordnung 1, 2, 3, 6 in jeweils

0002560

derselben Ebene bestimmt worden sind, kann daraus die zweidimensionale Absorptionsverteilung des Körpers in dieser Ebene berechnet werden. Häufig interessiert jedoch nicht nur die Absorptionsverteilung in einer Ebene des Körpers, sondern in mehreren aufeinanderfolgenden Ebenen. Diese kann am einfachsten dadurch erhalten werden, dass nach jeder Serie von Messungen, bei der der Körper nacheinander um etwa 180° gegenüber der Anordnung gedreht wird, der Körper senkrecht zur Zeichenebene in Fig. 1 verschoben wird. Statt dessen kann auch durch entsprechende Ausbildung des Kollimators 2 ein zweidimensionaler Fächer von Strahlenbündeln 5 erzeugt werden. d.h. eine Anzahl ebener Fächer von Strahlenbündeln übereinander, so dass die Absorptionsverteilung in mehreren Ebenen des Körpers gleichzeitig bestimmt werden kann. Bei der Anordnung in Fig. 1 sind dann mehrere Detektoranordnungen 6 übereinander angeordnet, und jede ist mit einem eigenen Verstärker 7 und einer eigenen, getrennten Verarbeitungsanordnung 8 versehen, so dass die Intensitäten und damit die Absorptionswerte jeweils für alle übereinanderliegenden Strahlenbündel gleichzeitig erzeugt werden. Wenn die Verarbeitungsanordnung 8 eine genügend grosse Verarbeitungsgeschwindigkeit besitzt, kann diese auch für alle übereinanderliegenden Detektorelemente 6 verwendet werden, deren Signale im Zeitmultiplex verarbeitet werden. Dies ist besonders zweckmässig für die in Fig. 2 dargestellte Blende, bei der dann mehrere spaltförmige Kollimatoren 2 übereinander hinter der Blende angeordnet sind, denn bei Drehung der Blende werden die Strahlenbündel der verschiedenen Kollimatoren für die unterschiedlichen Untersuchungsebenen zu verschiedenen Zeiten durch das Sektorende gesperrt. Um die Strahlenbündel eines Kollimators bzw. einer Ebene möglichst alle zum selben Zeitpunkt zu sperren, ist es dann günstiger, die Blende grösser zu wählen und das Verhältnis von Innenradius zu Aussenradius der Sektoren grösser, d.h. näher an 1 zu wählen. In Prinzip kann die in Fig. 2 dargestellte Blende auch für den Fall des zweidimensionalen Fächers

von Strahlenbündeln verwendet werden. Auch die in Fig.3 dargestellte Blende kann entsprechend verwendet werden, wenn die einzelnen Sektoren in radialer Richtung so lang ausgebildet werden, dass sie mehrere spaltförmige Kollimatoren übereinander überdecken.

Es kann aber auch für den gesamten zweidimensionalen Fächer von Strahlenbündeln ein einziges Detektorelement verwendet werden, so dass dann auch nur ein Verstärker 7 und eine Verarbeitungsanordnung 8 vorhanden ist. In diesem Falle muss der zweidimensionale Fächer von Strahlenbündeln 5 mit den Funktionen eines zweidimensionalen, vollständigen, linear unabhängigen Funktionssystems moduliert werden, und die Verarbeitungsanordnung 8 muss mit den diskreten Signalwerten eine zweidimensionale Rücktransformation durchführen. Beispiel für eine Blende 3 mit einer zweidimensionalen Walsh-Funktion ist in Fig. 4 dargestellt. Darin ist der Sektor S21 ganz durchlässig. Von dem Sektor S22 ist nur die obere Hälfte durchlässig, während der untere, gestrichelt gezeichnete Teil undurchlässig ist. Von dem Sektor S23 ist ebenfalls nur die Hälfte durchlässig, wobei nun aber die Trennlinie in radialer Richtung verläuft. Im Sektor S24 sind die durchlässige und undurchlässigen Teile nach Art eines Schachbrettmusters angeordnet.

Die Rücktransformation bei Verwendung einer derartigen Blende erfolgt dadurch, dass die vier dem Detektor 6 aufeinanderfolgend entnommenen Signalwerte in einer Weise mit Werten multipliziert werden, die der nachfolgend angegebenen zweiseitigen Matrixmultiplikation entspricht:

$$[B] = c \begin{pmatrix} 1 & 1 \\ 1 & -1 \end{pmatrix} [b] \begin{pmatrix} 1 & 1 \\ 1 & -1 \end{pmatrix}$$

Darin ist B die Bildmatrix, d.h. die zweidimensionale Verteilung der Intensitätswerte bei unmodulierter Strahlung, b ist die transformierte Matrix, die sich aus den matrixförmig angeordneten, dem Detektorelement aufeinanderfolgend entnommenen Werten ergibt,

0002560

und C ist ein geeigneter Normierungsfaktor. Allgemein
gilt für die Rücktransformation, wenn die transformierte
Matrix durch Modulation mittels entsprechend ausgebildeter
Blenden auf folgende Weise von der Bildmatrix abhängt.

$$[b] = [T] [B] [T]^+$$

wobei T und $T^+$ zueinander inverse Matrizen sind, die
die modulierenden Funktionen bestimmen, dass daraus die
Bildmatrix auf folgende Weise gewonnen wird:

$$[B] = [T]^+ [b] [T]$$

wenn es sich um eine separable Transformation handelt,
bei der nacheinander die Zeilen durch linksseitige und
die Spalten durch rechtsseitige Matrixmultiplikation
transformiert werden können. Bei nicht separabelen
Funktionssystemen muss statt dessen eine Tensoroperation
höherer Ordnung durchgeführt werden, die auch bei
separablen Systemen möglich ist, jedoch mehr Rechenoperationen erfordert.

Die Massnahmen zur Verringerung der Strahlungsdosis durch Weglassen des Gleichanteils und Verringern
der Amplitude der Sequenzen höherer Ordnung können auch
grundsätzlich bei zweidimensionalen Funktionssystemen
angewendet werden, wenn diese eine Transformation in
den Sequenzbereich bewirken.

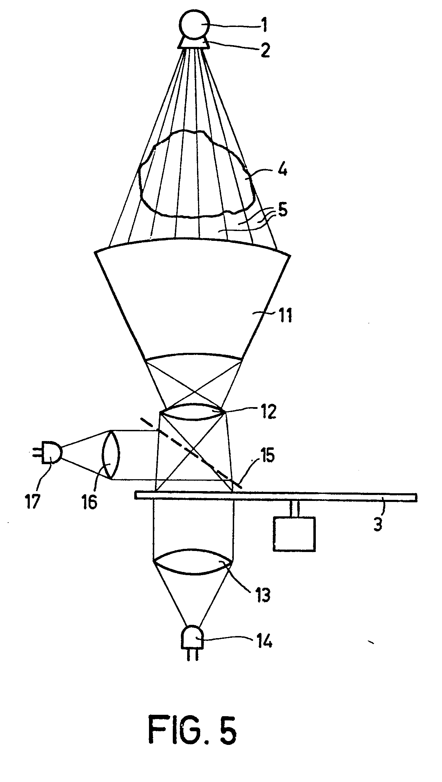

Bei dem in Fig. 1 dargestellten Beispiel ist
die Blende 3 zwischen der Quelle 1 der Strahlung und
dem zu untersuchenden Körper 4 angeordnet. Grundsätzlich
kann die Blende 3 jedoch auch zwischen dem Körper 4
und der Detektoranordnung 6 angeordnet sein. Dies ist
in Fig. 5 dargestellt, in der mit der Fig. 1 übereinstimmende Elemente mit den gleichen Bezugszeichen
versehen sind.

Die Strahlenbündel 5, die den Körper durchdrungen haben, treffen hier jedoch zunächst auf eine
Bildwandlerröhre 11 auf, die die von der Strahlungsquelle 1,

0002560

die wieder durch eine Röntgenröhre gebildet sein möge, ausgesandte Strahlung mit hoher Empfindlichkeit in sichtbares Licht umwandelt. Dabei erfolgt zunächst keine Veränderung der in der Strahlung enthaltenen Information, sondern nur eine Veränderung von deren Darstellung.

Die von der Bildwandlerröhre 11 abgegebene sichtbare Strahlung wird durch eine Optik 12 auf die rotierende Blende 3 abgebildet, die ebenso wie die in Fig. 3 bis 5 dargestellten Beispiele aufgebaut sein kann. Die von der Blende 3 durchgelassene Strahlung wird von der Optik 13 auf das Detektorelement 14 gesammelt, das beispielsweise eine Photodiode ist. Das Signal dieser Photodiode 14 wird in gleicher Weise weiter behandelt wie das Signal der Detektoranordnung 6 in Fig. 1.

Da in diesem Falle von der Strahlungsmenge, die den Körper 4 durchdrungen hat, infolge der Blende 3 nur ein Teil auf der Photodiode 14 wirksam wird, wird der Körper 4 mit einer entsprechend grösseren Strahlungsdosis belastet. Um auch die von der Blende 3 nicht durchlassene Strahlung auszunutzen, ist die Blende 3 auf der der Optik 12 zugewandten Seite reflektierend ausgebildet, und zwischen der Blende 3 und der Optik 12 ist ein halbdurchlässiger Spiegel 15 angeordnet. Dadurch wird die von der Blende 3 nicht durchgelassene Strahlung reflektiert, durch den Spiegel 15 abgelenkt und von der Optik 16 auf eine weitere Photodiode 17 gesammelt. Die beiden Photodioden 14 und 17 liefern dadurch zueinander komplentäre Signale, die zur Vergrösserung des Nutzsignals voneinander subtrahiert werden können. Im Falle von Walsh-Funktionen kann beispielsweise der Photodiode 14 der Wert "1" und der Photodiode 17 der Wert "-1" zugeordnet werden.

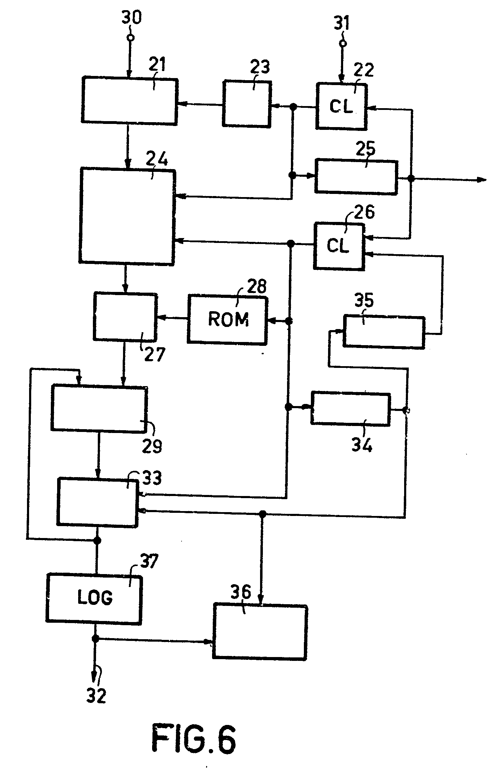

Eine Ausbildung der Verabeitungsanordnung 8 aus Fig. 1 ist in Fig. 6 dargestellt. Bei der Verwendung vershobener Funktionen beim Modulieren, wie bei Verwendung von Walsh-Funktionen in Zusammenhang mit Fig.2 beschrieben, werden die vom Verstärker 7 in Fig. 1 gelieferten Signale über den Eingang 30 einer Subtrahierstufe 21 zugeführt, die von den Signalwerten einen dazu
gehörenden im Messvorgang ermittelten Verschiebungswert
subtrahiert, der in dem Speicher 23 gespeichert ist.
Die auf diese Weise erzeugten unmodifizierten Walsh-
Spektralwerte der Intensitätsfunktion werden in einen
Speicher 24 eingeschrieben, wobei das Einschreiben durch
den Takt eines Taktgenerators 22 gesteuert wird.

Der Taktgenerator 12 wird durch ein am Eingang
31 zugeführtes Synchronisiersignal gestartet, das abhängig
von der Drehung der Blende 3 auf bekannte Weise mit
Positionsdetektor 10 z.B. foto-elektrisch oder magnetisch
erzeugt werden kann, und zwar immer zu dem Zeitpunkt,
wenn bei der in Fig. 2 dargestellten Blende das Ende
des Sektors 1 gerade vor dem Kollimator 2 angelangt ist.
Es kann auch am Ende jedes Sektors ein Signal erzeugt
werden, so dass der Taktgenerator 22 kein selbstschwingender Oszillator, sondern praktisch nur ein Pulsverstärker sein muss. Anderenfalls erzeugt der Taktgenerator 22 bei einem einmaligen Synchronisiersignal je
Blendenumdrehung anschliessend selbsttätig Impulse in
einem solchen Abstand, die dem Vorbeilaufen der einzelnen
Sektoren der Blende vor dem Kollimator entsprechen.

Der Speicher 24 kann ein Speicher mit wahlfreiem Zugriff, ein Silo-Speicher oder ein Schieberegister sein. Ferner versorgt der Taktgenerator 22 einen
ersten Steuerzähler 25 mit Zähltakten, der nach Zählung
der vorgegebenen Anzahl von Taktsignalen und damit Signalwerten am Eingang 30 entsprechend der Anzahl verschiedener
Funktionen bzw. Blendensektoren ein Ausgangssignal abgibt,
das den Taktgenerator 22 sperrt und einen weiteren
Taktgenerator 26 startet. Gleichzeitig löst dieses Signal
eine Drehung des Körpers 4 in Fig. 1 gegenüber der Messanordnung zur Einstellung der nächsten Messposition
aus, und während dieser Einstellzeit werden die Synchronisiersignale von der Blende 3 unterdrückt.

Während dieser Zeit erzeugt der Taktgenerator 26
Taktsignale, die die vorher in den Speicher 24 einge-

0002560

schriebenen Signalwerte in gleicher Reihenfolge nacheinander ausgelesen und einer Multiplizieranordnung 27 zuführen. Gleichzeitig steuern diese Taktsignale einen Festwertspeicher 28 an, der nacheinander zeilenweise die Werte der Rücktransformationsmatrix abgibt. Wenn beim Modulieren Walsh-Funktionen verwendet werden, wie vorher beschrieben wurde, enthält diese Rücktransformationsmatrix nur die Werte 1 oder -1, so dass der Multiplizierer 27 nur eine Vorzeichenumkehr durchführen muss.

Die Ausgangswerte des Multipliziereres 27 werden einer Addierstufe 29 zugeführt, deren Ausgang mit einem Register 33 verbunden ist dessen Ausgang wiederum auch auf den anderen Eingang der Addierstufe 29 führt. Die Addierstufe 29 wirkt zusammen mit dem Register 33 dadurch als Akkumulator. Falls die Addierstufe 29 einen Steuereingang zum Umschalten auf Subtraktion besitzt, ist es zweckmässig, bei Verwendung von Walsh-Funktionen beim Modulieren diesen Steuereingang direkt vom Ausgang des Speichers 18 anzusteuern, so dass dann der Multiplizierer 27 entfallen kann.

Die vom Taktgenerator 26 erzeugten Taktsignale schreiben mit jedem Auslesen des nächsten Wertes aus dem Speicher 24 den vorhergehend verarbeiteten Wert in das Register 33 ein. Ferner wird ein Zähler 34 um eine Stellung weitergeschaltet, der nach Zählung einer der Anzahl verwendeter Funktionen beim Modulieren entsprechender Taktsignale ein Ausgangssignal abgibt, das anzeigt, dass der nun vorhandene Inhalt des Registers 33 einen rücktransformierten Intensitätswerte eines Strahlenbündels darstellt. Aus diesem Intensitätswert wird in bekannter Weise durch Logarithmieren in der Stufe 37 der Absorptionswert gewonnen. Dieser Absorptionswert wird über den Ausgang 32 herausgeführt und einer Anordnung für die Rekonstruktion der zweidimensionalen Absorptionsverteilung zugeführt. Statt dessen kann dieser Registerinhalt auch in einen weiteren Speicher 36 eingeschrieben werden, der alle Absorptionswerte der ganzen Folge von Messpositionen aufnimmt und später zur Bestimmung der Absorptionsverteilung in der Fläche wieder abgibt.

Anschliessend wird das Register 33 mit diesem Ausgangssignal gelöscht, damit nun der nächste rücktransformierte
Intensitätswert bzw. Absorptionswert bestimmt werden kann.

Mit den folgenden Taktimpulsen des Taktgenerators
26 werden die im Speicher 24 enthaltenen Werte erneut
ausgelesen, so dass dieser Speicher für nicht zerstörendes
Auslesen geeignet sein muss. Gleichzeitig werden aus dem
Speicher 28 die Signalwerte der nächsten Zeile der Rücktransformationsmatrix ausgelesen, und durch akkumulierende
Addition entsteht schliesslich im Register 33 der nächste
rücktransformierte Intensitätswert und am Ausgang 32
der nächste Absorptionswert, wenn das nächste Ausgangssignal des Zählers 34 erscheint.

Auf diese Weise werden nacheinander alle
Absorptionswerte des Körpers 4 längs der einzelnen
Strahlenbündel zurückgewonnen, und deren Anzahl ist
gleich der Anzahl der Funktionen in dem beim Modulieren
verwendeten Funktionssystem. Daher wird das Ausgangssignal des Zählers 34 einem weiteren Zähler 35 zugeleitet,
der ebenfalls bei einer Zählerstellung entsprechend der
Anzahl der Funktionen ein Ausgangssignal abgibt, das den
Taktgenerator 26 wieder stillsetzt, da die Rücktransformation abgeschlossen ist. Sobald der Körper 4 in
Fig. 1 relativ zur Messanordnung die neue Messposition
erreicht hat, wird das Synchronisiersignal von der Blende
3 wieder freigegeben, und die nächste Folge von Signalwerten entsprechend der transformierten Intensitätsfolge
erscheint am Eingang 30 und wird wieder schritthaltend
in den Speicher 24 eingeschrieben. Die Rücktransformation
der im Speicher 24 enthaltenen Werte muss also in der
Zeit abgeschlossen sein, bis der Körper 4 seine neue
Messposition erreicht hat, und entsprechend ist die
Frequenz des Taktgenerators 26 zu wählen.

"PATENTANSPRÜCHE:"

1.       Anordnung zur Bestimmung der Absorptionsverteilung eines Körpers mittels einer im wesentlichen
punktförmigen Quelle zur Erzeugung von den Körper durchdringenden Strahlung und einer Detektoranordnung, die
die den Körper durchdrungene Strahlung aufnimmt und in
ein Detektor-Signal umwandelt, und einer Verarbeitungsanordnung, die aus den Detektorsignalen elektrische
Signale erzeugt, die die Absorption des Körpers langs
benachbarter Strahlenbündel angeben, dadurch gekennzeichnet, dass die Detektoranordnung für jeweils eine
Gruppe benachbarter Strahlenbündel (5) nur ein Detektorelement enthält, dass eine Modulationsanordnung (3)
die Intensität der Strahlenbündel nach Zeitfunktionen
moduliert, die jeweils für die von einem Detektorelement
aufgenommenen Strahlenbündel ein vollständiges, linear
unabhängiges Funktionssystem bilden, bei dem jede Funktion
eindeutig einem Strahlenbündel zugeordnet ist, wobei die
Funktionen positive Funktionswerte beinhalten und dass
die Verarbeitungsanordnung (7,8) aus den Detektorsignalen
eine Anzahl vorzugsweise in etwa gleichen zeitlichen
Abständen liegende diskrete Signalwerte entnimmt und
speichert, wobei die Anzahl der diskreten Signalwerte
gleich der Anzahl der Funktionen des zugehörigen
Funktionssystems ist, und durch Multiplikation der diskreten Signalwerte mit einer Matrix von Werten, die
zu der Matrix von Werten invers ist, die die Funktionswerte der beim Modulieren verwendeten Funktionen an den
der Entnahme der diskreten Signalwerte entsprechenden
Stellen darstellen, und nachfolgende Logarithmierung
die die Absorptionswerte angebenden Signalwerte erzeugen.

2.        Anordnung nach Anspruch 1, dadurch gekennzeichnet, dass die Modulationsanordnung (3) jedes
Strahlenbündel (5) mit nur diskreten Funktionswerten
der zugehörigen Funktionen während jeweils einer vorgegebenen Zeitdauer moduliert, wobei die Anzahl der
diskreten Funktionswerte gleich der Anzahl der verwendeten
Funktionen des zugehörigen Funktionssystems ist und die
Funktionswerte in etwa gleichen zeitlichen Abständen aus
den Funktionen entsprechend den Stellen der Entnahme der
diskreten Signalwerte in der Verarbeitungsanordnung (7,8)
entnommen sind.

3.        Anordnung nach Anspruch 2, dadurch gekennzeichnet, dass die Modulationsanordnung (3) die Strahlenbündel (5) mit den zugehörigen Funktionswerten derjenigen
Funktionen, die die höheren Sequenzen der durch die Folge
der Absorptionswerte längs benachbarter Strahlenbündel
gebildeten Absorptionsfunktion erzeugen, während kürzerer
Zeitdauer moduliert als mit den Funktionswerten der übrigen
Funktionen und während der restlichen Zeit diese Strahlenbündel ganz sperrt.

4.        Anordnung nach Anspruch 1 oder einem der
folgenden, dadurch gekennzeichnet, dass in den Funktionssystemen die Funktion weggelassen ist, die den Gleichanteil, d.h. den Mittelwert der Absorptionswerte für die
Strahlenbündel (5) eines Detektorelementes, liefert.

5.        Anordnung nach Anspruch 1 oder einem der
folgenden, dadurch gekennzeichnet, dass für alle Strahlenbündel (5) nur ein Detektorelement (6) vorhanden ist.

6.        Anordnung nach Anspruch 1 oder einem der
folgenden, dadurch gekennzeichnet, dass die Strahlenbündel
(5) einen zweidimensionalen Fächer bilden und jeweils für nur in einer Dimension benachbarte Strahlenbündel ein Detektorelement (6) vorgesehen ist.

7.     Anordnung nach Anspruch 6, dadurch gekennzeichnet, dass für alle Strahlenbündel (5) nur ein Detektorelement (6) vorhanden ist und die Modulationsanordnung (3) die Strahlenbündel nach Zeitfunktionen moduliert, die ein zweidimensionales, vollständiges, linear unabhängiges Funktionssystem bilden.

8.     Anordnung nach Anspruch 1 oder einem der folgenden, dadurch gekennzeichnet, dass die Modulationsanordnung (3) eine mechanisch bewegte Blende zwischen der Quelle (1) der Strahlung und der Detektoranordnung (6) ist, die entsprechend den Funktionen der Funktionssysteme für die Strahlung durchlässige bzw. undurchlässige Bereiche besitzt.

9.     Anordnung nach Anspruch 8, dadurch gekennzeichnet, dass die Blende (3) aus einer oder mehreren rotierenden Scheiben besteht.

10.     Anordnung nach Anspruch 8 oder 9, dadurch gekennzeichnet, dass von der Bewegung der Blende (3) Synchronisiersignale zum Steuern der Entnahme der diskreten Signalwerte in der Verarbeitungsanordnung (7,8) abgeleitet sind.

11.     Anordnung nach Anspruch 9 oder einem der folgenden, dadurch gekennzeichnet, dass die Blende (3) zwischen dem Körper (4) und der Detektoranordnung (6) angeordnet ist und zwischen der Blende (3) und der Detektoranordnung (6) eine Bildwandleranordnung (11) vorgesehen ist.

FIG.1

1-Ⅴ- PHD   77-155

FIG.2

**FIG.3**

**FIG.4**

FIG. 5

5/5

FIG.6

# EUROPÄISCHER RECHERCHENBERICHT

**Europäisches Patentamt**

Nummer der Anmeldung

EP 78 20 0377

## EINSCHLÄGIGE DOKUMENTE

KLASSIFIKATION DER ANMELDUNG (Int.Cl.²)

| Kategorie | Kennzeichnung des Dokuments mit Angabe, soweit erforderlich, der maßgeblichen Teile | betrifft Anspruch |
|---|---|---|
| | <u>US - A - 3 944 833</u> (G.N. HOUNS-FIELD/ EMI LTD)<br><br>* Zusammenfassung; Spalte 7, Zeile 62 - Spalte 8, Zeile 63; Spalte 10, Zeile 28 - Spalte 11, Zeile 38; Spalte 12, Zeilen 58-61; Abbildungen 6a,7,10 *<br><br>-- | 1,6,8, 9 |
| - | <u>US - A - 3 965 353</u> (A. MACOVSKI)<br><br>* Zusammenfassung; Spalte 6, Zeilen 31-49; Abbildung 4 *<br><br>-- | 1,8 |
| | <u>DE - A - 2 452 166</u> (PHILIPS PATENTVERWALTUNG GmbH)<br><br>* Seite 3, Zeile 14 - Seite 7, Zeile 13; Abbildungen *<br><br>-- | 1,7, 8,11 |
| | <u>FR - A - 1 184 655</u> (N.V. PHILIPS' GLOEILAMPENFABRIEKEN)<br><br>* Seite 2, rechte Spalte, Zeile 5 - Seite 3, linke Spalte, Zeile 52; Abbildung 1 *<br><br>-- | 1,8,9 |
| | <u>FR - A - 2 340 555</u> (N.V. PHILIPS' GLOEILAMPENFABRIEKEN)<br><br>* Seite 8, Zeile 30 - Seite 9, Zeile 19; Seite 11, Zeile 25 - Seite 12, Zeile 16; Abbildungen 2-4c *<br><br>-- | 1,8 |
| | <u>FR - A - 2 344 032</u> (N.V. PHILIPS' GLOEILAMPENFABRIEKEN)<br><br>* Seite 8, Zeile 13 - Seite 11, Zeile 26; Abbildungen 1,3 * | 1,8 |

**KLASSIFIKATION DER ANMELDUNG (Int.Cl.²)**

A 61 B 6/02
6/06

**RECHERCHIERTE SACHGEBIETE (Int.Cl.²)**

A 61 B 6/02
6/06

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 09-03-1979 | RIEB |

EPA form 1503.1 06.78